# EUROPÄISCHE PATENTSCHRIFT

(11) **EP 0 734 245 B1**
(45) Veröffentlichungstag und Bekanntmachung des Hinweises auf die Patenterteilung: **07.10.1998**
(21) Anmeldenummer: 95904436.3
(22) Anmeldetag: 08.12.1994
(51) Int. Cl.: A61K 7/32

(54) **DEODORIERENDE ZUBEREITUNGEN**
DEODORANT PREPARATIONS
PREPARATIONS DEODORANTES

(30) Priorität: 17.12.1993 DE 4343264
(43) Veröffentlichungstag der Anmeldung: 02.10.1996
(73) Patentinhaber: Henkel Kommanditgesellschaft auf Aktien, 40191 Düsseldorf (DE)
(72) Erfinder: WACHTER, Rolf, D-40595 Düsseldorf (DE); MAURER, Karl-Heinz, D-40699 Erkrath (DE); TESMANN, Holger, D-41363 Jüchen (DE)
(86) Internationale Anmeldenummer: EP9404084
(87) Internationale Veröffentlichungsnummer: WO9516429

(56) Entgegenhaltungen:
- EP-A- 0 006 234
- EP-A- 0 347 664
- DE-A- 2 721 297

## Beschreibung

Die Erfindung betrifft die Verwendung von fettlöslichen Hydroxycarbonsäureestern als deodorierende Wirkstoffe in topischen Zubereitungen zur Unterdrückung von Körpergeruch.

Der störende Geruch, der die Transpiration des Menschen begleitet, wird hauptsächlich durch die bakterielle Zersetzung des zunächst geruchlosen Schweißes auf der Haut und in der Kleidung verursacht. Man hat daher bisher zur Unterdrückung des Körpergeruchs entweder schweißhemmende Präparate oder antimikrobielle Stoffe eingesetzt. Auch geruchsüberdeckende Parfüms und geruchsabsorbierende Stoffe, z.B. Polymere, wurden vorgeschlagen, weisen jedoch nur eine begrenzte Wirksamkeit auf. Sowohl die schweißhemmenden, adstringierenden Stoffe als auch die antimikrobiellen Stoffe bringen bei regelmäßiger Anwendung auf der Haut die Gefahr von Reizungen und Unverträglichkeiten mit sich.

Es besteht daher ein ständiges Bedürfnis, dermatologisch sicher anwendbare, gut verträgliche Deodorantwirkstoffe zu finden, die auch bei langzeitiger Anwendung die natürliche Hautflora nicht stören.

Aus Cosmetics & Toileteries, Vol. 95, July 1980, Seiten 48 - 50 sind bestimmte Ester von Hydroxycarbonsäuren, wie z.B. das Ethyllactat oder das Triethylcitrat als nicht-mikrobizide Deodorant-Wirkstoffe bekannt. Die Wirkung dieser Stoffe wurde auf eine Esterase-Inhibierung aufgrund der bei der enzymatischen Hydrolyse durch Esterasen aus Bakterien der Hautflora freigesetzten Säure zurückgeführt.

Es wurde nun gefunden, daß fettlösliche Partialester von mehrbasischen Hydroxycarbonsäuren in pH-stabilisierten (auf pH = 6 gepufferten) wäßrigen Medien eine erheblich höhere esterasehemmende Wirkung aufweisen als die genannten kurzkettigen Hydroxycarbonsäureester. Die fettlöslichen Partialester mehrbasischer Hydroxyverbindungen eignen sich darüber hinaus aufgrund ihrer problemlosen Hautverträglichkeit besonders gut als deodorierende Wirkstoffe in topischen Zubereitungen.

Gegenstand der Erfindung ist die Verwendung von fettlöslichen Hydroxycarbonsäureestern, die erhältlich sind durch Veresterung von mehrbasischen, gegebenenfalls acetylierten Hydroxycarbonsäuren mit 3 bis 6 C-Atomen durch Umsetzung mit Fettsäuren mit 12 - 30 C-Atomen, Fettsäureestern aus C₁₂-C₂₂-Fettsäuren und C₁-C₄-Alkoholen oder mit Hydroxylverbindungen aus der Gruppe
(a) Fettalkohole mit 12 - 30 C-Atomen
(b) Fettsäureester aus C₁₂-C₂₂-Fettsäuren und Polyolen mit 2 - 6 C-Atomen und 2 - 6 Hydroxylgruppen
(c) Epoxyalkane mit 12 - 30 C-Atomen
(d) Alkylglycoside der Formel R¹O (C₆H₁₀O)ₓ-H, in der R¹ eine Alkylgruppe mit 8 - 16 C-Atomen und x der mittlere Oligomerisationsgrad des Glycosidrestes (C₆H₁₀O) einen Wert von 1 - 4 hat und
(e) Anlagerungsprodukte von 1 - 8 Mol Ethylenoxid an die Hydroxylverbindungen (a) bis (d)
als deodorierende Wirkstoffe in topischen Zubereitungen. Bevorzugt werden als fettlösliche Ester die Partialester der Hydroxycarbonsäuren mit Hydroxylverbindungen (b) bis (e) oder deren Salze eingesetzt.

Unter Partialestern sollen solche Ester verstanden werden, die wenigstens eine der Carboxylgruppen der mehrbasischen Hydroxycarbonsäure noch unverestert enthalten.

Als mehrbasische Hydroxycarbonsäuren eignen sich z.B. Äpfelsäure, Schleimsäure, Tartronsäure, Weinsäure bzw. Traubensäure (DL-Weinsäure) oder Mesoweinsäure und Citronensäure. Die fettlöslichen Partialester der Citronensäure und Weinsäure bzw. der Acetyl-Citronensäure oder der Mono- oder Diacetylweinsäure sind bevorzugt. Der Einsatz der acetylierten Hydroxycarbonsäuren ist insbesondere bei der Weinsäure bevorzugt, um die Selbstkondensation der Hydroxycarbonsäuren weitgehend zu verhindern.

Die lipophilen Partialester werden nach bekannten Verfahren durch Umsetzung mit Fettsäuren oder Alkoholen mit 12 - 30 C-Atomen erhalten. Bei der Umsetzung mit Fettsäuren kommt es zur Veresterung der Hydroxylgruppe der Hydroxycarbonsäuren. Durch Umsetzung mit Hydroxylverbindungen wird bevorzugt eine der Carboxylgruppen der Hydroxycarbonsäuren verestert. Geeignete Fettsäuren sind z.B. Laurinsäure, Palmitinsäure, Stearinsäure, Ölsäure und Erucasäure oder technische Gemische solcher Fettsäuren, z.B. Kokosfettsäure-Schnitte mit 12 - 18 C-Atomen oder Palmkernölfettsäure, Talgfettsäure oder Palmitin-/ Stearinsäure-Gemische. Geeignete Alkohole sind z.B. Cetyl-/Stearylalkohol, Kokosfettalkohol-Schnitte mit 12 - 18 oder 12 - 14 C-Atomen, geh. Talgalkohol, Behenylalkohol, Triacontanol, Sterole wie z.B. Cholesterol oder Phytosterol und Tocopherole.

Durch Umsetzung der mehrbasischen Hydroxycarbonsäuren mit Fettsäuren und einfachen Estern kommt es zu Umesterungs-Reaktionen unter Veresterung der Hydroxylgruppe der Hydroxycarbonsäuren, bei Umsetzung mit Fettsäure-Polyestern mit freien Hydroxylgruppen kommt es überwiegend zur partiellen Veresterung der Carboxylfunktion der Hydroxycarbonsäuren, in sehr geringem Umfange zur Umesterung mit der Hydroxylgruppe der Hydroxycarbonsäuren.

Bevorzugt geeignete Fettsäure-Polyolester sind z.B. Glycerinmonostearat, geh. Talgfettsäure-monoglycerid, Sorbitan-monooleat, Sorbitansesquistearat, Propylenglycol-monopalmitat, Pentaerythrit-dimyristat, Diethylenglycol-monostearat. Bevorzugt werden als fettlösliche Partialester die Veresterungsprodukte aus gegebenenfalls acetylierter Citronensäure oder Weinsäure und Fettsäuremonoglyceriden mit 12 bis 18 C-Atomen eingesetzt.

Die Herstellung der fettlöslichen Partialester ist literaturbekannt und z.B. in DE 14 68 454 beschrieben. Aus dieser Druckschrift ist die Eignung solcher Ester als Antioxidantien bekannt. Aus FR 1 437 366 ist auch die gute Hautverträglichkeit solcher fettlöslicher Hydroxycarbonsäure-Partialester bekannt. Einige geeignete Produkte sind als Emulgatoren und Hilfsstoffe für die Lebensmittelindustrie im Handel. Hierfür werden als Beispiele genannt:
- Lamegin ZE 309^{(R)}:: Ein Citronensäureester des Monoglycerids der gehärteten Talgfettsäure (Säurezahl: ca. 70, Verseifungszahl: ca. 305)
- Lamegin DW 8000^{(R)}:: Ein Ester der Diacetylweinsäure mit dem Monoglycerid der gehärteten Talgfettsäure (Säurezahl: ca. 100, Verseifungszahl: ca. 510)

Die erfindungsgemäß geeigneten fettlöslichen Hydroxycarbonsäure-Partialester werden in den deodorierenden kosmetischen Zubereitungen bevorzugt als alleinige deodorierende Komponente eingesetzt. Ein weiterer Gegenstand der Erfindung sind daher deodorierende kosmetische Zubereitungen, die einen Gehalt von ca. 0,5 bis 10 Gew.-% an lipophilen Hydroxycarbonsäure-Partialestern, die erhältlich sind durch Umsetzung von mehrbasischen, gegebenenfalls acetylierten Hydroxycarbonsäuren mit 2 - 4 C-Atomen mit Fettsäuren oder Alkoholen mit 12 - 30 C-Atomen oder mit Fettsäureestern aus C₁₂-C₂₂-Fettsäuren und Alkoholen mit 1 - 4 C-Atomen oder Polyolen mit 2 - 6 C-Atomen und 2 - 6 Hydroxylgruppen, in einem wäßrigen, emulsionsförmigen, wäßrig-alkoholischen oder wasserfreien Träger aufweisen. Als fettlösliche Hydroxycarbonsäure-Partialester sind dabei bevorzugt die Ester der Citronensäure oder Weinsäure enthalten. Besonders hohe esterasehemmende Wirkung haben auch die Ester der Citronensäure und Weinsäure mit Tocopherolen im Molverhältnis 1 : 1 bis 1 : 2. Als Tocopherole eignen sich natürliche Tocopherole (α-Tocopherol, β-Tocopherol oder γ-Tocopherol) sowie Mischungen dieser Stoffe und synthetische Analoga.

Um die besonderen Vorteile dieser nicht-antimikrobiellen Deodorantien zu nutzen, sollten die erfindungsgemäßen Zubereitungen bevorzugt frei von antimikrobiellen Stoffen sein. Andererseits kann ein Zusatz antibakterieller oder antitranspiratorischer Komponenten den deodorierenden Effekt weiter steigern.

Die als Träger geeigneten, wäßrigen, emulsionsförmigen, wäßrig-alkoholischen oder wasserfreien Zubereitungen können flüssig, pastös oder fest sein. Es kann sich dabei um flüssige Lotionen oder niedrigviskose Emulsionen handeln, die aus Pumpzerstäubern oder mit Hilfe von Aerosol-Treibgasen auf die Haut gebracht werden. Es kann sich auch um verdickte Lotionen oder Emulsionen handeln, die aus Roll-on-Spendebehältern aufgetragen werden. Schließlich können die erfindungsgemäßen Zubereitungen auch als deodorierende Salben oder Cremes, als deodorierende Stiftpräparate oder deodorierende Seifen formuliert werden.

Die Einarbeitung der fettlöslichen Hydroxycarbonsäure-Partialester erfolgt dabei auf übliche Weise durch Solubilisation mit niederen Alkoholen wie Ethanol oder Isopropanol und üblichen Lösungsvermittlern oder durch Emulgierung unter Verwendung bekannter Emulgatoren. Bevorzugte Emulgatoren zur Einarbeitung der erfindungsgemäß geeigneten Hydroxycarbonsäure-Partialester in wäßrige und wäßrig-alkoholische Zubereitungen sind dabei die Anlagerungsprodukte von Ethylenoxid (EO) an lineare Fettalkohole, an Fettsäuren, an Fettsäurepartialglyceride oder Fettsäure-Sorbitanester. Zur stabilen Emulgierung werden bevorzugt weitere, lipophile Coemulgatoren verwendet, bevorzugt Cetyl-/Stearylalkohol oder Glycerinmonostearat. Darüber hinaus können die erfindungsgemäßen deodorierenden Zubereitungen alle für die jeweilige Applikationsform erforderlichen Hilfsmittel und Zusätze enthalten, z.B. kosmetische Öle, Fette, Wachse, Glycerin, Propylenglycol, wasserlösliche Polymere, organophile Tone sowie Farb- und Duftstoffe, Komplexbildner, Antioxidantien, Antitranspirantien (z.B. Al-Hydroxychlorid) und Konservierungsmittel. Auch kosmetische Wirkstoffe, z.B. zur Straffung, Feuchthaltung oder Rückfettung der Haut können zugesetzt werden.

Die folgenden Beispiele sollen den Erfindungsgegenstand näher erläutern:

### Beispiele

### 1. Bestimmung der Esterase-Hemmwirkung der erfindungsgemäßen Partialester

Die Bestimmung der Esterase-Wirkung erfolgte in einem Testsystem, das Schweineleber-Esterase in einer wäßrigen, auf pH = 6 gepufferten Lösung von p-Nitrophenyl-butyrat enthielt. Die Esterase-Wirkung zeigte sich in einer Spaltung des p-Nitrophenyl-butyrats unter Freisetzung des p-Nitrophenols, dessen Extinktionskoeffizient im Photometer bei 410 nm (25°C) über 3 Minuten verfolgt wurde und eine Berechnung des molaren Substrat-Umsatzes als Maß für die Enzymaktivität ermöglichte.

Die Esterase-Hemmwirkung der Prüfsubstanzen wurde als Esterase-Restaktivität nach 15-minütiger Einwirkzeit der Prüfsubstanzen auf die Esterase-Lösung analog den oben beschriebenen Verfahren ermittelt. Sie ist in den folgenden Tabellen in % der ungehemmten Esterase-Aktivität angegeben.

**Tabelle I**

| Prüfsubstanz | Esterase-Restaktivität bei Einsatz von | |
|---|---|---|
| | 1000 ppm | 5000 ppm |
| Ester aus Citronensäure und geh. Talgfettsäuremonoglycerid (Lamegin ZE 309) | 0 | 0 |
| Ester aus Diacetyl-Weinsäure und geh. Talgfettsäuremonoglycerid (Lamegin DW 8000) | 10 | 0 |
| Citronensäure-Tocopherol-Ester (1 : 2) | 1 | 0 |
| Triethylcitrat (Vergleich) | 84 % | 62 % |

**Tabelle II**

| Prüfsubstanz | Esterase-Restaktivität bei Einsatz von | | |
|---|---|---|---|
| | 100 ppm | 500 ppm | 5000 ppm |
| Veresterungsprodukt aus 1 Mol Weinsäure und 1 Mol Laurylalkohol | 17 | 0 | 0 |
| Veresterungsprodukt aus 1 Mol Zitronensäure und 2 Mol des Anlagerungsprodukts von 7 Mol Ethylenoxid an 1 Mol Lauryl/Myristyl(2/1)-Alkohol | - | 90 | 32 |
| Zitronensäure-tri-2-hydroxyalkyl(C₁₂-C₁₈)-ester (aus Zitronensäure mit 1,2-Epoxy-C₁₂-C₁₈-alkan) | - | 46 | 7 |
| Schleimsäure-dialkyl(C₈/C₁₀)-ester | - | 80 | 23 |

### 2. Anwendungsbeispiele

| 1. Roll-on-Deodorants | | | | |
|---|---|---|---|---|
| | a | b | c | d |
| Glycerin-(mono/di)-stearat/palmitat (ca. 50 % Monoglycerid, ca. 50 % Stearat) | 4 | 5 | 5 | 5 |
| Cetyl-/stearylalkohol + 12 EO | 1,5 | 1 | 1 | 1 |
| Cetyl-/stearylalkohol + 20 EO | 1,5 | 2 | 2 | 2 |
| Cetyl-palmitat | - | 1 | 1 | 1 |
| Cetyl-/stearylalkohol | - | 1,5 | 1,5 | 1,5 |
| 2-Octyl-dodecanol | 3,5 | 2 | 2 | 2 |
| Di-n-octylether | - | 2 | 2 | 2 |
| α-Tocopherol-Citronensäureester (2:1) | 2,5 | 3 | - | - |
| geh. Talgfettsäuremonoglycerid-Citronensäureester (Lamegin^{(R)}ZE 309) | - | - | 3 | - |
| geh. Talgfettsäuremonoglycerid-Diacetyl-Weinsäureester (Lamegin^{(R)}DW 8000) | - | - | - | 3 |
| Glycerin | 5 | 3 | 3 | 3 |
| Carbopol^{(R)}980, (2 %ig in H₂O) (Na-Polyacrylat-Gel) | 15 | - | - | - |
| Ethanol (98,8 %ig) | 10 | - | - | - |
| Wasser, NaOH bis pH = 6 | 57 | 79,5 | 79,5 | 79,5 |

## Patentansprüche

1. Verwendung fettlöslicher Hydroxycarbonsäureestern, die erhältlich sind durch Veresterung von mehrbasischen, gegebenenfalls acetylierten Hydroxycarbonsäuren mit 3 bis 6 C-Atomen durch Umsetzung mit Fettsäuren mit 12 - 30 C-Atomen, Fettsäureestern aus C₁₂-C₂₂-Fettsäuren und C₁-C₄-Alkoholen oder mit Hydroxylverbindungen aus der Gruppe
(a) Fettalkohole mit 12 - 30 C-Atomen
(b) Fettsäureester aus C₁₂-C₂₂-Fettsäuren und Polyolen mit 2 - 6 C-Atomen und 2 - 6 Hydroxylgruppen
(c) Epoxyalkane mit 12 - 30 C-Atomen
(d) Alkylglycoside der Formel R¹O (C₆H₁₀O)ₓ-H, in der R¹ eine Alkylgruppe mit 8 - 16 C-Atomen und x der mittlere Oligomerisationsgrad des Glycosidrestes (C₆H₁₀O) einen Wert von 1 - 4 hat und
(e) Anlagerungsprodukte von 1 - 8 Mol Ethylenoxid an die Hydroxylverbindungen (a) bis (d)
als deodorierende Wirkstoffe in topischen Zubereitungen zur Unterdrückung von Körpergeruch.

2. Verwendung nach Anspruch 1, dadurch gekennzeichnet, daß als fettlösliche Ester die Partialester der Hydroxycarbonsäuren mit Hydroxylverbindungen (a) bis (e) oder deren Salze eingesetzt werden.

## Claims

1. The use of fat-soluble hydroxycarboxylic acid esters obtainable by esterification of polybasic, optionally acetylated hydroxycarboxylic acids containing 3 to 6 carbon atoms by reaction with fatty acids containing 12 to 30 carbon atoms, fatty acid esters of C₁₂₋₂₂ fatty acids and C₁₋₄ alcohols or with hydroxyl compounds from the group consisting of
(a) fatty alcohols containing 12 to 30 carbon atoms,
(b) fatty acid esters of C₁₂₋₂₂ fatty acids and polyols containing 2 to 6 carbon atoms and 2 to 6 hydroxyl groups,
(c) epoxyalkanes containing 12 to 30 carbon atoms,
(d) alkyl glycosides corresponding to the formula R¹O(C₆H₁₀O)ₓ-H, in which R¹ is an alkyl group containing 8 to 16 carbon atoms and x, the average degree of oligomerization of the glycoside unit (C₆H₁₀O), has a value of 1 to 4 and
(e) adducts of 1 to 8 moles of ethylene oxide with the hydroxyl compounds (a) to (d)
as deodorizing agents in topical preparations for suppressing body odour.

2. The use claimed in claim 1, characterized in that the partial esters of hydroxycarboxylic acids with hydroxyl compounds (a) to (e) or salts thereof are used as the fat-soluble esters.

## Revendications

1. Utilisation d'esters d'hydroxyacides carboxyliques solubles dans les matières grasses, gu'on peut obtenir par estérification d'hydroxyacides carboxyliques polybasiques acétylés le cas échéant avec 3 à 6 atomes de C par réaction avec des acides gras de 12-30 atomes de C, des esters d'acides gras d'acides gras en C₁₂-C₂₂ et d'alcools C₁-C₄ ou avec des composés hydroxylés du groupe :
a) alcools gras de 12-30 atomes de C,
b) esters d'acide gras des acides gras C₁₂-C₂₂ et des polyols de 2-6 atomes de C et 2-6 groupes hydroxyles,
c) époxyalcanes avec 12-30 atomes de C,
d) alkylglycosides de formule R¹O(C₆H₁₀O)ₓ-H où R¹ est un groupe alkyle de 8-16 atomes de C et x est le degré moyen d'oligomérisation du reste glycoside (C₆H₁₀O) et a une valeur de 1-4 et,
e) produit d'addition de 1-8 moles d'oxyde d'éthylène sur les composés hydroxylés (a) à (d), comme principes actifs désodorisants dans des compositions topiques pour supprimer l'odeur corporelle.

2. Utilisation selon la revendication 1,
caractérisée en ce qu'
on utilise comme esters solubles dans les matières grasses les esters partiels des hydroxyacides carboxyliques avec des composés hydroxylés (a) à (e) ou leurs sels.
